# FASCICULE DE BREVET EUROPEEN

(11) **EP 4 169 485 B1**
(45) Date de publication et mention de la délivrance du brevet: **03.07.2024**
(21) Numéro de dépôt: 21203844.2
(22) Date de dépôt: 21.10.2021
(51) Int. Cl.: A61F 2/30

(54) **IMPLANT À ANCRAGE OSSEUX ET PROCÉDÉ DE FABRICATION D'UN TEL IMPLANT**
KNOCHENVERANKERTES IMPLANTAT UND HERSTELLUNGSVERFAHREN EINES SOLCHEN IMPLANTATS
IMPLANT WITH BONE ANCHOR AND METHOD FOR MANUFACTURING SUCH AN IMPLANT

(43) Date de publication de la demande: 26.04.2023
(73) Titulaire: ANTHOGYR, 74700 Sallanches (FR)
(72) Inventeur: COURTOIS, Nicolas, 74190 PASSY (FR); ADOLFSSON, Erik, 412 67 GÖTEBORG (SE)
(74) Mandataire: Cabinet Poncet

(56) Documents cités:
- EP-A1- 2 022 447
- US-A1- 2017 001 920
- US-A1- 2020 368 028

## Description

### DOMAINE TECHNIQUE DE L'INVENTION

La présente invention concerne le domaine des implants à ancrage osseux destinés à être au moins en partie enfouis et retenus dans un os d'un être vivant tel qu'un humain ou un animal. L'art antérieur le plus proche est le document US 2020/368028 A1, qui définit le préambule de la revendication 1.

De tels implants à ancrage osseux sont couramment utilisés afin de notamment consolider, ou remplacer une partie d'os ou d'articulation.

Parmi les implants à ancrage osseux, on connaît par exemple les implants dentaires, destinés à être enfouis dans l'os maxillaire ou mandibulaire d'un patient afin de recevoir et porter une prothèse dentaire dans le cadre d'une restauration dentaire.

On connaît aussi les implants orthopédiques, parmi lesquels on trouve par exemple les prothèses orthopédiques de hanche. Une prothèse de hanche comprend généralement deux parties implantables constituant une articulation à rotule, à savoir une partie femelle destinée à remplacer le cotyle naturel de la hanche et une partie mâle destinée à remplacer la tête du fémur.

La partie mâle de l'articulation comporte généralement une tige dite tige fémorale, destinée à être insérée et ancrée dans le canal médullaire du fémur, et dont l'extrémité proximale se raccorde par un col à une tête sphérique destinée à pénétrer dans la partie femelle.

La partie femelle de l'articulation, qui doit remplacer le cotyle naturel de la hanche, et que l'on désignera globalement par l'appellation cotyle, comprend habituellement une cupule d'insertion hémisphérique, qui vient se loger dans une cavité cotyloïdienne préparée de l'os du bassin.

Dans tous les cas d'implants à ancrage osseux, tels qu'un implant dentaire ou un implant orthopédique, il importe que l'implant soit retenu à long terme selon une force de rétention suffisamment élevée (stabilité à long terme) dans l'os dans lequel il est implanté.

Il importe par ailleurs que, après son insertion dans l'os, l'implant ait au plus vite une bonne stabilité dite « stabilité primaire » de façon à pouvoir être mis sous charge au plus vite dans le but de raccourcir le temps de traitement du patient.

Par ailleurs, il importe de faciliter et/ou accélérer l'ancrage d'implants dans l'os de personnes bénéficiant de capacités d'ostéointégration moindres ou dégradées, telles que les personnes diabétiques ou fumeuses de tabac.

Les fabricants d'implants à ancrage osseux travaillent ainsi depuis longtemps sur la surface des implants destinée à venir au contact de l'os en vue de favoriser leur ostéointégration.

Il a ainsi été développé un procédé de traitement de surface d'implants (notamment en titane ou alliage de titane) appelé BCP (« Biphasic Calcium Phosphate » en anglais) consistant à sabler la surface extérieure de l'implant avec une poudre qui est un mélange d'hydroxyapatite (HA) et de β-TCP (phosphate tricalcique bêta) puis à la soumettre à une attaque par l'acide nitrique (HNO₃), à un rinçage et à un séchage.

Il a également été mis au point un procédé de traitement de surface d'implants (notamment en titane ou alliage de titane) appelé SLA (« Sandblasted Large grit Acid etched » en anglais) consistant à sabler la surface extérieure de l'implant, puis à lui faire subir un double mordançage à l'acide chlorhydrique et à l'acide sulfurique.

La société STRAUMANN a amélioré le procédé SLA en ajoutant une étape de séchage sous atmosphère azotée et une conservation hermétique dans une solution de chlorure de sodium isotonique. Il s'agit d'un procédé appelé commercialement SLActive ^{®}.

La société ASTRA TECH a de son côté proposé un procédé de traitement de surface d'implants (notamment en titane ou alliage de titane) commercialement appelé « Osseo Speed » consistant à projeter sur la surface de l'implant des particules de dioxyde de titane, puis à modifier chimiquement la surface de l'implant par de l'acide fluorhydrique.

La société BIOMET 3i a proposé un procédé de traitement de surface d'implants (notamment en titane ou alliage de titane) commercialement appelé « Nano Tite » consistant à former sur la surface de l'implant une couche discontinue au moyen de grains de 10 à 100 nm de phosphate de calcium déposés de façon discrète. Cette couche procure une topographie de surface complexe censée optimiser les avantages biologiques du phosphate de calcium.

La société NOBEL BIOCARE a proposé un procédé de traitement de surface d'implants (notamment en titane ou alliage de titane) commercialement appelé TiUnite ^{®} consistant à former une surface poreuse en dioxyde de titane par électro-anodisation à partir d'une surface d'implant usinée.

Tous ces procédés connus offrent toutefois des performances qui méritent d'être largement améliorées.

Par ailleurs, tous ces procédés connus ont été développés pour des implants métalliques (notamment en titane ou alliage de titane). Or, il se développe de nos jours un intérêt particulier pour des implants en céramique pour des raisons esthétiques (notamment dans le domaine dentaire) et des raisons de biocompatibilité. En effet, les implants métalliques (en titane ou alliages de titane par exemple) présentent une coloration très foncée qui contraste fortement avec les couleurs naturelles des dents. Au contraire, les couleurs de matériaux céramiques peuvent correspondre de façon très proche aux couleurs naturelles des dents. Par ailleurs les métaux et alliages de métaux présentent inéluctablement des impuretés dans leur composition chimique, lesquelles impuretés peuvent provoquer une réaction de rejet par l'organisme.

Il a ainsi été recherché des procédés de traitement de surface d'implants permettant aussi de favoriser, d'accélérer et/ou d'augmenter l'ostéointégration d'implants, en particulier pour des implants en céramique. Le document EP 1 982 670 B1 décrit un tel procédé pour un implant en céramique.

### EXPOSE DE L'INVENTION

Un problème proposé par la présente invention est de fournir un implant à ostéointégration augmentée et/ou accélérée et/ou facilitée.

Simultanément, l'invention vise à fournir un implant en céramique à ostéointégration augmentée et/ou accélérée et/ou facilitée.

Pour atteindre ces objets ainsi que d'autres, l'invention propose un implant destiné à être au moins en partie enfoui dans un os selon une partie d'implant présentant une surface endo-osseuse ; selon l'invention, ladite surface endo-osseuse comprend au moins une zone ayant une topographie de surface présentant :
- un paramètre de courbure moyenne arithmétique des pics (Spc) inférieur ou égal à 1 µm⁻¹,
- un paramètre de densité des pics (Spd) supérieur ou égal à 0,020 µm⁻².

La demanderesse a constaté de façon tout à fait surprenante et inattendue qu'une topographie de surface présentant de tels paramètres Spc et Spd procure une ostéointégration nettement améliorée.

Cette amélioration a été constatée lors d'essais qui ont notamment montré :
- que la force nécessaire pour extraire l'implant hors de l'os par traction au bout de 8 semaines est nettement augmentée, ce qui traduit une amélioration de la stabilité à long terme de l'implant, et/ou
- que la force nécessaire pour extraire l'implant hors de l'os par traction au bout de seulement 4 semaines est aussi nettement augmentée, ce qui traduit une accélération et/ou facilitation de l'ostéointégration et donc une amélioration de la stabilité primaire de l'implant, et/ou - qu'une grande proportion de la surface extérieure de l'implant se retrouve plus rapidement au contact de l'os.

Ces résultats laissent en outre envisager qu'est facilité et/ou accéléré l'ancrage d'implants dans l'os de personnes bénéficiant de capacités d'ostéointégration moindres ou dégradées, telles que les personnes diabétiques ou fumeuses de tabac.

De préférence, pour des résultats encore meilleurs, le rapport de la courbure moyenne arithmétique des pics (Spc) sur la densité des pics (Spd) peut être compris dans l'intervalle [5 ; 50].

Avantageusement, le corps de l'implant peut être en céramique, en métal ou en alliage de métaux.

De préférence, l'implant peut comporter une couche extérieure poreuse ayant une épaisseur comprise entre 1 et 5 µm. Une telle épaisseur n'est pas trop élevée, afin de limiter les risques que la couche extérieure poreuse soit friable, et afin de faciliter une décontamination efficace en cas de péri-implantite. Cette épaisseur n'est pas non plus trop faible, et ce afin de conserver une topographie efficace.

Avantageusement, pour des résultats encore meilleurs, le paramètre de densité des pics (Spd) peut être inférieur ou égal à 0,5 µm⁻².

Selon un autre aspect de la présente invention, il est proposé un procédé de fabrication d'un implant tel que décrit précédemment, comportant les étapes successives suivantes :
a) fournir un corps d'implant comprenant une surface endo-osseuse destinée à être enfouie dans un os,
b) fournir une solution d'un matériau organique dans un solvant, ladite solution comprenant un matériau particulaire en suspension,
c) appliquer la solution sur une zone de la surface endo-osseuse du corps d'implant,
d) évaporer le solvant,
e) chauffer le corps d'implant à une température de traitement, la température et la durée de traitement étant choisies suffisantes pour éliminer le matériau organique et insuffisantes pour faire fondre le matériau particulaire et le matériau constituant le corps d'implant.

Un tel procédé est simple à mettre en oeuvre de façon industrielle et peu onéreux.

Sans que la demanderesse puisse être liée par une quelconque théorie, il semblerait que :
- après évaporation du solvant, le matériau organique et le matériau particulaire restent par adhérence sur la surface du corps d'implant, le matériau organique contribuant à maintenir des espaces entre les grains du matériau particulaire ;
- l'élimination du matériau organique lors de l'étape e) mène à l'obtention d'une topographie de surface comprenant des pics (de matériau particulaire) et de creux (aux endroits où était le matériau organique avant élimination thermique).

Lors de l'étape e), la température et la durée de traitement choisies permettent de limiter les risques de dégradation (par fusion totale ou partielle) des pics créés en surface de l'implant grâce au matériau particulaire.

Avantageusement, pour de bons résultats en termes d'ostéointégration, avant l'étape e), on peut répéter les étapes c) et d) plusieurs fois, de préférence au moins 50 fois. La répétition permet d'ériger de façon progressive la topographie de surface.

De préférence, le matériau organique peut être un polymère, de préférence un polyéthylène glycol, encore de préférence un polyéthylène glycol à poids moléculaire d'environ 4 000 g/mol. Il s'agit ainsi de matériaux organiques simples, facilement disponibles et peu onéreux.

Avantageusement, pour préparer la solution de l'étape b), on utilise un volume du matériau organique qui peut être 1 à 20 fois, de préférence 1 à 10 fois, de façon encore préférée 1 à 6 fois, plus élevé que le volume de matériau particulaire. Il a en effet été constaté que de tels rapports permettaient l'obtention d'une topographie de surface satisfaisante.

Avantageusement, le matériau particulaire est une céramique en poudre, de préférence de la zircone, ou du métal en poudre, de préférence du titane ou un alliage de titane. On utilisera avantageusement une céramique en poudre pour créer une topographie de surface sur la surface extérieure d'un implant en céramique. On utilisera avantageusement du métal en poudre pour créer une topographie de surface sur la surface extérieure d'un implant métallique.

De préférence, lors de l'étape c), la solution peut être appliquée sur le corps d'implant par pulvérisation. Un tel mode d'application s'est révélé très approprié pour une solution comprenant un matériau particulaire en suspension.

Avantageusement, dans la solution, le matériau particulaire peut représenter 0,1 % à 10 % du volume de la solution, de préférence 0,5 % à 1 %.

De préférence, le matériau particulaire peut présenter une taille médiane de particules inférieure ou égale à 1 µm. La mesure des tailles des particules et de leur distribution est réalisée selon la norme ISO 13320, par exemple au moyen d'une diffractométrie laser en voie liquide sur un équipement de mesure commercialisé sous la dénomination « MASTERSIZER 3000 » par la société MALVERN PANALYTICAL.

Selon un mode de réalisation avantageux, on peut prévoir que :
- le corps d'implant est en céramique, de préférence en zircone, encore de préférence en zircone yttriée ou cériée,
- le matériau particulaire est une poudre de céramique, de préférence de zircone, encore de préférence en zircone yttriée ou ceriée,
- le matériau organique est du polyéthylène glycol.

Avantageusement, lors de l'étape e), on peut chauffer le corps d'implant à une température de traitement comprise entre 600°C et 1 600°C, de préférence entre 1 100°C et 1 600°C, et encore de préférence entre 1 300°C et 1 600°C. Une telle température de traitement s'avère avantageuse pour les corps d'implant en céramique. Une température minimale suffisante permet de bien éliminer le matériau organique et de conférer des formes et dimensions stables au corps d'implant et à sa topographie de surface. Une température maximale inférieure à 1 600°C permet de limiter les risques de dégradation des propriétés mécaniques de la céramique du corps d'implant.

De préférence, le corps d'implant peut être en une céramique, en un métal ou en un alliage de métaux, dont les dimensions, au cours de l'étape e), ne varient pas de plus de 3 à 5 %. On limite ainsi les risques d'une désolidarisation accidentelle de la couche externe (formant la topographie de surface) du corps de l'implant.

Avantageusement, le corps d'implant peut être en une céramique, en un métal ou en un alliage de métaux, dont la masse volumique est d'au moins 95 % de sa masse volumique théorique. On favorise ainsi la formation de pics en surface du corps d'implant par l'application de la solution comprenant un matériau particulaire en suspension. En dessous du rapport de 95 %, on a constaté que le matériau du corps d'implant a généralement une structure poreuse qui a trop tendance à absorber la solution comprenant un matériau particulaire en suspension, ce qui nuit à la formation d'une topographie de surface satisfaisante.

La présente invention est applicable aux implants dentaires.

La présente invention est également applicable à tous les autres implants à ancrage osseux, tels que les implants orthopédiques.

### DESCRIPTION SOMMAIRE DES DESSINS

D'autres objets, caractéristiques et avantages de la présente invention ressortiront de la description suivante de modes de réalisation particuliers, faite en relation avec les figures jointes, parmi lesquelles :
[Fig.1] La figure 1 est un schéma illustrant les différentes étapes d'un mode de réalisation particulier de procédé permettant d'obtenir un implant selon la présente invention ;
[Fig.2] La figure 2 est une vue à l'échelle micrométrique d'un exemple de topographie de surface obtenue par le procédé de la figure 1 sur un corps d'implant ; et
[Fig.3] La figure 3 est une vue de différents implants utilisés lors de tests effectués par la demanderesse.

### DESCRIPTION DES MODES DE REALISATION PREFERES

Lorsque des références numériques identiques sont utilisées dans plusieurs figures, modes de réalisation ou variantes de l'invention, ces références numériques désignent des éléments identiques ou similaires dans chacun(e) des figures, modes de réalisation ou variantes.

### Procédé de fabrication

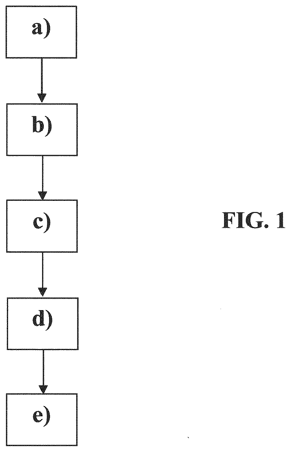

Sur la figure 1 est schématiquement illustré un exemple de procédé de fabrication permettant d'obtenir un implant selon la présente invention.

Dans cet exemple, le procédé de fabrication comporte les étapes successives suivantes :
a) fournir un corps d'implant comprenant une surface endo-osseuse destinée à être enfouie dans un os,
b) fournir une solution d'un matériau organique dans un solvant, ladite solution comprenant un matériau particulaire en suspension,
c) appliquer la solution sur une zone de la surface endo-osseuse du corps d'implant,
d) évaporer le solvant,
e) chauffer le corps d'implant à une température de traitement, la température et la durée de traitement étant choisies suffisantes pour éliminer le matériau organique et insuffisantes pour faire fondre le matériau particulaire et le matériau constituant le corps d'implant.

Avant l'étape e), on répète les étapes c) et d) plusieurs fois, de préférence au moins 50 fois. En d'autres termes, l'étape e) n'est réalisée qu'une fois, après qu'aient été répétées les étapes c) et d) un nombre de fois suffisant. Réaliser 50 fois les étapes c) et d) a mené à d'excellentes topographies.

Le matériau organique est un polymère, de préférence un polyéthylène glycol, encore de préférence un polyéthylène glycol à poids moléculaire d'environ 4 000 g/mol.

Pour préparer la solution de l'étape b), on utilise un volume du matériau organique qui est 1 à 20 fois, de préférence 1 à 10 fois, de façon encore préférée 1 à 6 fois, plus élevé que le volume de matériau particulaire.

Le matériau particulaire est une céramique en poudre, de préférence de la zircone, si le corps d'implant est en céramique. En alternative, le matériau particulaire est du métal en poudre, de préférence du titane ou un alliage de titane, si le corps d'implant est en métal.

Lors de l'étape c), la solution est appliquée sur le corps d'implant par pulvérisation.

Dans la solution, le matériau particulaire représente 0,1 % à 10 % du volume de la solution, de préférence 0,5 % à 1 %.

Le matériau particulaire présente une taille médiane de particules inférieure ou égale à 1 µm.

Lors de l'étape e), on chauffe le corps d'implant à une température de traitement comprise entre 600°C et 1 600°C.

Le corps d'implant est en une céramique, en un métal ou en un alliage de métaux, dont les dimensions, au cours de l'étape e), ne varient pas de plus de 3 à 5 %.

Le corps d'implant est en une céramique, en un métal ou en un alliage de métaux, dont la masse volumique est d'au moins 95 % de sa masse volumique théorique.

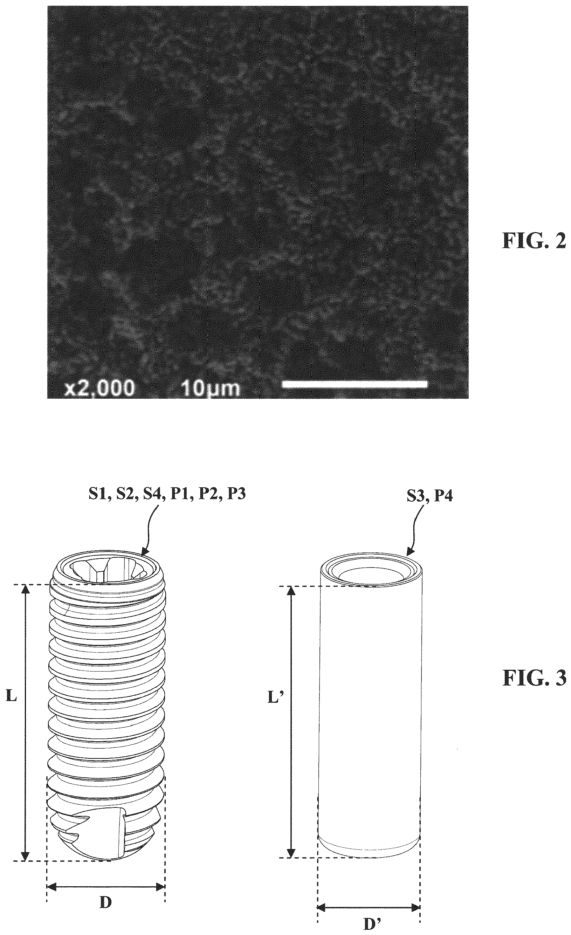

Ce procédé selon l'invention a mené à l'obtention de l'exemple de topographie de surface illustrée sur la figure 2. Sur cette figure, on observe que la topographie de surface obtenue se présente sous la forme d'une couche superficielle de 1 à 5 µm à densité réduite où les particules de céramique sont réparties de façon quasi-homogène, avec une faible distance entre elles. Les particules de céramique forment des pics entre lesquels apparaissent des creux laissés par le matériau organique qui a été éliminé lors de l'étape e). Il n'a pas été observé de pores réels.

### Formes et matériaux des implants testés

Sur la figure 3 sont illustrés plusieurs implants S1 à S4 et P1 à P4 utilisés lors de tests.

Les implants S1, S2, S4 et P1 à P3 présentent une forme cylindrique ayant une longueur L de 12 mm et avec une section circulaire ayant un diamètre D de 4,6 mm. Ils sont pourvus d'un même filetage extérieur sur leur surface latérale pour permettre leur insertion par vissage dans un trou ménagé dans un os.

Les implants P4 et S3 présentent une forme cylindrique ayant une longueur L' de 12 mm et une section circulaire ayant un diamètre D' de 4,2 mm. Ils sont dépourvus de filetage extérieur sur leur surface latérale et sont destinés à être insérés par impaction dans un trou ménagé dans un os.

Les implants S1 à S4 et P1 sont en céramique, plus particulièrement en un mélange de zircone ceriée et d'alumine.

Les implants P2 à P4 sont en métal. L'implant P2 est plus particulièrement en un alliage de titane Ti13Zr. Les implants P3 et P4 sont plus particulièrement en un alliage de titane Ti6Al4V ELI.

### Implants à topographie de surface selon l'invention

Les implants « S » (S1 à S4) sont des implants selon la présente invention, sur la surface extérieure desquels a été réalisée une topographie de surface selon le procédé de la présente invention illustré sur la figure 1.

Lors de l'étape c), il a été appliqué par pulvérisation sur la surface extérieure des implants S1 à S4 une solution d'un matériau organique (du polyéthylène glycol à poids moléculaire d'environ 4 000 g/mol) dans un solvant, ladite solution comprenant un matériau particulaire en suspension (constitué d'un mélange de zircone ceriée et d'alumine).

Le matériau particulaire était ainsi une poudre céramique mise en suspension. Dans celle-ci, 10 % des particules en suspension avaient une taille inférieure à 0,15 µm, 50 % des particules en suspension avaient une taille inférieure à 0,4 µm et 90 % des particules en suspension avaient une taille inférieure à 2 µm. La mesure de cette distribution en taille des particules du matériau particulaire a été réalisée selon la norme ISO 13320 par diffractométrie laser en voie liquide sur un équipement de mesure commercialisé sous la dénomination « MASTERSIZER 3000 » par la société MALVERN PANALYTICAL.

Les étapes c) et d) ont été répétées environ 50 fois avant que soit réalisée l'étape e) à une même température de traitement comprise entre 1 350°C et 1 450°C pour chaque implant.

Pour l'implant S1, on a utilisé un volume du matériau organique qui était 2 fois plus élevé que le volume de matériau particulaire. Pour les implants S2 et S3, on a utilisé un volume du matériau organique qui était 4 fois plus élevé que le volume de matériau particulaire. Pour l'implant S4, on a utilisé un volume du matériau organique qui était 6 fois plus élevé que le volume de matériau particulaire.

Les paramètres Spc, Spd et les rapports Spc/Spd des topographies de surface des implants S1 à S4 sont donnés par le Tableau 1.

La topographie des surfaces a été examinée quantitativement sur une surface de 351 µm × 264 µm à l'aide d'un microscope confocal 3D (S-Neox, Sensofar Tech S.L, Terrassa, Espagne). Un objectif confocal avec un grossissement de 50 fois, donnant une résolution latérale de 0,26 µm a été utilisé. La quantité totale de points non mesurés était inférieure à 5%.

L'ensemble des opérations a été appliqué au résultat de la mesure à l'aide du logiciel SensoMAP (SensoMAP, version 7.4.8803, SENSOFAR TECH, Terrassa, Espagne), utilisant la base Mountains Map^{®} (édité par Digital Surf, Besançon, France), incluant le module Grains et Particules et le module de caractérisation de surface avancée 3D.

Les paramètres Spc et Spd décrits dans la norme ISO25178 ont été extraits des mesures qui ont été effectuées par :
- débruitage (filtre médian 3 × 3),
- application d'un opérateur F - suppression de la forme (polynôme du 5ème degré),
- remplissage des points non mesurés avec une forme lisse calculée à partir des points voisins,
- application d'un filtre S (0,8 µm × 0,8 µm, filtre gaussien),
- application d'un filtre L (25 µm × 25 µm, filtre gaussien robuste),
- seuillage pour ne garder que les 95 % de données centrées autour du plan médian.

**[Tableau 1] Paramètres Spc, Spd et rapports Spc/Spd des topographies de surface des implants S1 à S4**

| **Implant** | **Spc (µm⁻¹)** | | **Spd (µm⁻²)** | | **Spc/Spd (µm)** | |
|---|---|---|---|---|---|---|
| | Moyenne sur 9 mesures | Ecart-type | Moyenne sur 9 mesures | Ecart-type | Moyenne sur 9 mesures | Ecart-type |
| **S1** | 0,521 | 0,147 | 0,040 | 0,006 | 13,251 | 3,940 |
| **S2** | 0,573 | 0,119 | 0,027 | 0,008 | 21,526 | 3,116 |
| **S3** | 0,459 | 0,036 | 0,021 | 0,001 | 22,390 | 2,910 |
| **S4** | 0,644 | 0,166 | 0,020 | 0,007 | 33,172 | 4,676 |

### Implants à topographie de surface selon l'art antérieur

Les implants « P » (P1 à P4) sont des implants sur la surface extérieure desquels a été réalisée une topographie de surface selon des procédés conventionnels connus. Les implants P1 à P4 sont ainsi des implants de contrôle permettant de se rendre compte des effets procurés par la présente invention aux implants S1 à S4.

La surface extérieure de l'implant P1 (en céramique) a subi un sablage avec des grains d'alumine (Al₂O₃) suivi d'une double attaque à l'acide fluorhydrique (HF) et à l'acide nitrique (HNO₃).

La surface extérieure de l'implant P2 (en métal) a subi un traitement de type SLA avec un sablage suivi d'un double mordançage à l'acide chlorhydrique (HCl) et à l'acide sulfurique (H₂SO₄).

Les surfaces extérieures des implants P3 et P4 (en métal) ont subi un traitement de type BCP.

Les paramètres Spc, Spd et les rapports Spc/Spd des topographies de surface des implants P1 à P4 ont été mesurés comme pour les implants S1 à S4, et sont donnés par le Tableau 2.

**[Tableau 2] Paramètres Spc, Spd et rapports Spc/Spd des topographies de surface des implants P1 à P4**

| **Implant** | **Spc (µm⁻¹)** | | **Spd (µm⁻²)** | | **Spc/Spd (µm)** | |
|---|---|---|---|---|---|---|
| | Moyenne sur 9 mesures | Ecart-type | Moyenne sur 9 mesures | Ecart-type | Moyenne sur 9 mesures | Ecart-type |
| **P1** | 1,493 | 0,557 | 0,025 | 0,009 | 60,414 | 12,430 |
| **P2** | 3,501 | 0,877 | 0,013 | 0,003 | 275,970 | 84,871 |
| **P3** | 7,644 | 1,582 | 0,023 | 0,005 | 338,207 | 90,669 |
| **P4** | 6,056 | 0,558 | 0,015 | 0,001 | 400,514 | 44,251 |

### Extraction après implantation in vivo

Il a été procédé à l'insertion de douze implants S3 et de douze implants P4 dans les humérus de douze brebis distinctes. Chaque brebis a reçu un implant S3 et un implant P4, respectivement implantés dans son humérus droit ou gauche. A l'issue d'une période de 4 semaines après implantation, 6 brebis ont été sacrifiées pour procéder à l'extraction axiale des implants au moyen d'une machine de traction INSTRON munie d'un capteur d'effort. A l'issue d'une période de 8 semaines après implantation, les 6 brebis restantes ont été sacrifiées pour procéder à l'extraction axiale des implants au moyen de la même machine de traction INSTRON munie d'un capteur d'effort.

Les résultats sont donnés par le Tableau 3.

**[Tableau 3] Forces d'extraction des implants**

| **Temps d'étude** | **Implant** | **Force d'extraction (N)** | |
|---|---|---|---|
| | | **Moyenne sur 6 implants (N)** | **Ecart-type** |
| **4 semaines** | **S3** | 870 | 160 |
| | **P4** | 482 | 72 |
| **8 semaines** | **S3** | 975 | 284 |
| | **P4** | 552 | 145 |

On observe ainsi que les implants S3 présentent une résistance à l'extraction nettement plus élevée que les implants P4, que ce soit à 4 semaines ou à 8 semaines. A 4 semaines, la résistance à l'extraction des implants S3 est environ 80 % supérieure à la résistance à l'extraction des implants P4. A 8 semaines, la résistance à l'extraction des implants S3 est environ 77 % supérieure à la résistance à l'extraction des implants P4. L'accroissement proportionnel entre 4 et 8 semaines est sensiblement identique entre les implants S3 (+12%) et P4 (+14%).

Ces résultats tendent à démontrer que les implants selon l'invention présentent une ostéointégration significativement plus précoce, et qu'à plus long terme l'ostéintégration est significativement meilleure.

### Mesure de la proportion de la surface extérieure de l'implant se trouvant au contact de l'os après implantation in vivo

Il a été procédé à l'insertion de douze de chacun des implants S1, S2 et S4 (soit 36 implants) et douze de chacun des implants P1, P2 et P3 (soit 36 implants) dans les fémurs de douze brebis distinctes. Chaque brebis avait ainsi 6 implants, à savoir 3 implants identiques implantés dans son fémur droit et 3 autres implants identiques dans son fémur gauche. A l'issue d'une période de 4 semaines après implantation, 6 brebis ont été sacrifiées pour procéder à l'évaluation du paramètre BIC (« Bone to Implant Contact » en anglais) permettant de déterminer la proportion de la surface extérieure de l'implant se trouvant au contact de l'os. A l'issue d'une période de 8 semaines après implantation, les 6 brebis restantes ont été sacrifiées pour procéder par tomographie à l'évaluation du paramètre BIC (« Bone to Implant Contact » en anglais) permettant de déterminer la proportion de la surface extérieure de l'implant se trouvant au contact de l'os.

Les résultats sont donnés par le Tableau 4.

**[Tableau 4] Proportion de la surface extérieure de l'implant se trouvant au contact de l'os (BIC)**

| **Temps d'étude** | **Implants** | **BIC %** | |
|---|---|---|---|
| | | **Moyenne sur 6 implants** | **Ecart-type** |
| **4 semaines** | **S1** | 79,6 | 7,1 |
| | **S2** | 73,8 | 2,5 |
| | **S4** | 70,3 | 9,1 |
| | **P1** | 42,4 | 18,4 |
| | **P2** | 55,8 | 11,3 |
| | **P3** | 68,6 | 8,2 |
| **8 semaines** | **S1** | 78,4 | 5,4 |
| | **S2** | 88,2 | 5,4 |
| | **S4** | 76 | 10,8 |
| | **P1** | 79,7 | 7,9 |
| | **P2** | 75,9 | 6,2 |
| | **P3** | 78,8 | 4,2 |

On observe ainsi que, à 4 semaines, les paramètres BIC respectifs des implants S1, S2 et S4 sont tous supérieurs (et même très nettement supérieurs en ce qui concerne les implants S1 et S2) aux paramètres BIC respectifs des implants P1, P2 et P3.

On observe aussi que, à 8 semaines, les paramètres BIC de tous les implants sont relativement proches. Les implants S2 montrent toutefois un paramètre BIC le plus élevé.

Ces résultats tendent à démontrer que les implants selon l'invention présentent une ostéointégration plus précoce que les implants ayant subi des traitements de surface conventionnels connus. L'ostéintégration est significativement meilleure pour les implants S2.

Finalement, les tests réalisés permettent de démontrer que la présente invention procure une ostéointégration augmentée et/ou accélérée et/ou facilitée, en particulier pour un implant ayant un corps d'implant en céramique.

Une partie des travaux relatifs à la présente invention a été réalisée dans le cadre du Consortium LONGLIFE et a reçu un support financier du septième programme-cadre de l'Union Européenne (FP7/2007-2013) sous le numéro d'agrément 280741.

La présente invention n'est pas limitée aux modes de réalisation qui ont été explicitement décrits, mais elle en inclut les diverses variantes et généralisations contenues dans le domaine des revendications ci-après.

## Revendications

1. - Implant destiné à être au moins en partie enfoui dans un os selon une partie d'implant présentant une surface endo-osseuse, **caractérisé en ce que** ladite surface endo-osseuse comprend au moins une zone ayant une topographie de surface présentant :
- un paramètre de courbure moyenne arithmétique des pics, à savoir le paramètre Spc tel que définit dans la norme ISO25178, inférieur ou égal à 1 µm⁻¹,
- un paramètre de densité des pics, à savoir le paramètre Spd tel que définit dans la norme ISO25178, supérieur ou égal à 0,020 µm⁻².

2. - Implant selon la revendication 1, le rapport de la courbure moyenne arithmétique des pics (Ope) sur la densité des pics étant compris dans l'intervalle [5 ; 50].

3. - Implant selon l'une des revendications 1 ou 2, le corps de l'implant étant en céramique, en métal ou en alliage de métaux.

4. - Implant selon l'une quelconque des revendications 1 à 3, l'implant comportant une couche extérieure poreuse ayant une épaisseur comprise entre 1 et 5 µm.

5. - Implant selon l'une quelconque des revendications 1 à 4, le paramètre de densité des pics étant inférieur ou égal à 0,5 um⁻².

6. - Procédé de fabrication d'un implant selon l'une quelconque des revendications 1 à 5, comportant les étapes successives suivantes :
a) fournir un corps d'implant comprenant une surface endo-osseuse destinée à être enfouie dans un os,
b) fournir une solution d'un matériau organique dans un solvant, ladite solution comprenant un matériau particulaire en suspension,
c) appliquer la solution sur une zone de la surface endo-osseuse du corps d'implant,
d) évaporer le solvant,
e) chauffer le corps d'implant à une température de traitement, la température et la durée de traitement étant choisies suffisantes pour éliminer le matériau organique et insuffisantes pour faire fondre le matériau particulaire et le matériau constituant le corps d'implant.

7. - Procédé selon la revendication 6, dans lequel, avant l'étape e), on répète les étapes c) et d) plusieurs fois, de préférence au moins 50 fois.

8. - Procédé selon l'une des revendications 6 ou 7, dans lequel le matériau organique est un polymère, de préférence un polyéthylène glycol, encore de préférence un polyéthylène glycol à poids moléculaire d'environ 4 000 g/mol.

9. - Procédé selon l'une quelconque des revendications 6 à 8, dans lequel, pour préparer la solution de l'étape b), on utilise un volume du matériau organique qui est 1 à 20 fois, de préférence 1 à 10 fois, de façon encore préférée 1 à 6 fois, plus élevé que le volume de matériau particulaire.

10. - Procédé selon l'une quelconque des revendications 6 à 9, dans lequel le matériau particulaire est une céramique en poudre, de préférence de la zircone, ou du métal en poudre, de préférence du titane ou un alliage de titane.

11. - Procédé selon l'une quelconque des revendications 6 à 10, dans lequel, lors de l'étape c), la solution est appliquée sur le corps d'implant par pulvérisation.

12. - Procédé selon l'une quelconque des revendications 6 à 11, dans lequel, dans la solution, le matériau particulaire représente 0,1 % à 10 % du volume de la solution, de préférence 0,5 % à 1 %.

13. - Procédé selon l'une quelconque des revendications 6 à 12, dans lequel le matériau particulaire présente une taille médiane de particules inférieure ou égale à 1 µm.

14. - Procédé selon l'une des revendications 6 à 13, dans lequel :
- le corps d'implant est en céramique, de préférence en zircone, encore de préférence en zircone yttriée ou cériée,
- le matériau particulaire est une poudre de céramique, de préférence de zircone, encore de préférence en zircone yttriée ou ceriée,
- le matériau organique est du polyéthylène glycol.

15. - Procédé selon l'une des revendications 6 à 14, dans lequel, lors de l'étape e), on chauffe le corps d'implant à une température de traitement comprise entre 600°C et 1 600°C, de préférence entre 1 100°C et 1 600°C, et encore de préférence entre 1 300°C et 1 600°C.

16. - Procédé selon l'une quelconque des revendications 6 à 15, dans lequel le corps d'implant est en une céramique, en un métal ou en un alliage de métaux, dont les dimensions, au cours de l'étape e), ne varient pas de plus de 3 à 5 %.

17. - Procédé selon l'une quelconque des revendications 6 à 16, dans lequel le corps d'implant est en une céramique, en un métal ou en un alliage de métaux, dont la masse volumique est d'au moins 95 % de sa masse volumique théorique.

## Patentansprüche

1. Implantat, das dazu bestimmt ist, mindestens teilweise entlang eines Implantatteils mit einer enossalen Oberfläche in einem Knochen eingebettet zu werden, **dadurch gekennzeichnet, dass** die besagte enossale Oberfläche mindestens eine Zone mit einer Oberflächentopografie umfasst, die aufweist:
- einen arithmetischen mittleren Krümmungsparameter der Peaks, d.h. den Parameter Spc gemäß der Definition in der ISO25178-Nomenklatur, der kleiner oder gleich 1 µm⁻¹ ist,
- einen Dichteparameter der Peaks, d.h. den Parameter Spd gemäß der Definition in der ISO25178-Nomenklatur, der größer oder gleich 0,020 µm⁻² ist.

2. Implantat nach Anspruch 1, wobei das Verhältnis der arithmetischen mittleren Krümmung der Peaks zur Dichte der Peaks in einem Intervall [5; 50] liegt.

3. Implantat nach einem der Ansprüche 1 oder 2, wobei der Körper des Implantats aus Keramik, Metall oder Metalllegierung ist.

4. Implantat nach einem der Ansprüche 1 bis 3, wobei das Implantat eine poröse Außenschicht aufweist, deren Dicke zwischen 1 und 5 µm beträgt.

5. Implantat nach einem der Ansprüche 1 bis 4, wobei der Dichteparameter der Peaks kleiner oder gleich 0,5 µm⁻² ist.

6. Verfahren zur Herstellung eines Implantats nach einem der Ansprüche 1 bis 5, aufweisend die folgenden aufeinanderfolgenden Schritte:
a) Bereitstellen eines Implantatkörpers, umfassend eine enossale Oberfläche, die dazu bestimmt ist, in einen Knochen eingebettet zu werden,
b) Bereitstellen einer Lösung eines organischen Materials in einem Lösungsmittel, wobei besagte Lösung suspendiertes Partikelmaterial umfasst,
c) Anwenden der Lösung auf eine Zone der enossalen Oberfläche des Implantatkörpers,
d) Verdampfen des Lösungsmittels,
e) Erhitzen des Implantatkörpers auf eine Behandlungstemperatur, wobei die Behandlungstemperatur und -dauer ausreichend gewählt werden, um das organische Material zu entfernen, und nicht ausreichend, um das Partikelmaterial und das Material, mit dem der Implantatkörper gebildet ist, zum Schmelzen zu bringen.

7. Verfahren nach Anspruch 6, wobei vor Schritt e) die Schritte c) und d) mehrmals, vorzugsweise mindestens 50-mal, wiederholt werden.

8. Verfahren nach einem der Ansprüche 6 oder 7, wobei das organische Material ein Polymer ist, vorzugsweise ein Polyethylenglykol, besonders bevorzugt ein Polyethylenglykol mit einem Molekulargewicht von etwa 4000 g/mol.

9. Verfahren nach einem der Ansprüche 6 bis 8, wobei zum Vorbereiten der Lösung von Schritt b) ein Volumen an organischem Material verwendet wird, das um das 1-bis 20-fache, vorzugsweise das 1- bis 10-fache, besonders bevorzugt das 1- bis 6-fache höher ist als das Volumen des Partikelmaterials.

10. Verfahren nach einem der Ansprüche 6 bis 9, wobei das Partikelmaterial eine pulverförmige Keramik, vorzugsweise Zirkonoxid, oder ein pulverförmiges Metall, vorzugsweise Titan oder eine Titanlegierung, ist.

11. Verfahren nach einem der Ansprüche 6 bis 10, wobei im Schritt c) die Lösung durch Zerstäuben auf den Implantatkörper angewendet wird.

12. Verfahren nach einem der Ansprüche 6 bis 11, wobei das Partikelmaterial in der Lösung 0,1 % bis 10 % des Volumens der Lösung ausmacht, vorzugsweise 0,5 % bis 1 %.

13. Verfahren nach einem der Ansprüche 6 bis 12, wobei das Partikelmaterial eine mediane Partikelgröße kleiner als oder gleich 1 µm aufweist.

14. Verfahren nach einem der Ansprüche 6 bis 13, wobei:
- der Implantatkörper aus Keramik, vorzugsweise aus Zirkonoxid, besonders bevorzugt aus Yttrium-haltigem oder Cer-haltigem Zirkonoxid, ist
- das Partikelmaterial ein Keramikpulver, vorzugsweise Zirkonoxid, besonders bevorzugt Yttrium-haltiges oder Cer-haltiges Zirkonoxid, ist,
- das organische Material mit Polyethylenglykol ist.

15. Verfahren nach einem der Ansprüche 6 bis 14, wobei beim Schritt e) der Implantatkörper auf eine Behandlungstemperatur zwischen 600°C und 1600°C, vorzugsweise zwischen 1100°C und 1600°C, besonders bevorzugt zwischen 1300°C und 1600°C erhitzt wird.

16. Verfahren nach einem der Ansprüche 6 bis 15, wobei der Implantatkörper aus einer Keramik, einem Metall oder einer Metalllegierung ist, wobei sich die Abmessungen während des Schritts e), um nicht mehr als 3 bis 5 % ändern.

17. Verfahren nach einem der Ansprüche 6 bis 16, wobei der Implantatkörper aus einer Keramik, einem Metall oder einer Metalllegierung ist, wobei das Massevolumen mindestens 95 % seines massentheoretischen Volumens ist.

## Claims

1. Implant intended to be at least partially implanted into a bone by means of an implant part having an endosseous surface, **characterized in that** said endosseous surface comprises at least one zone having a surface topography exhibiting:
- an arithmetic mean peak curvature parameter, namely the parameter Spc as defined in standard ISO25178, less than or equal to 1 µm⁻¹,
- a density of peaks parameter, namely the parameter Spd as defined in standard ISO25178, greater than or equal to 0.020 _{µm}⁻².

2. Implant according to Claim 1, the ratio of the arithmetic mean peak curvature to the density of peaks being comprised in the interval [5; 50].

3. Implant according to one of Claims 1 and 2, the body of the implant being made of ceramic, of metal, or of metal alloy.

4. Implant according to any one of Claims 1 to 3, the implant comprising a porous exterior layer having a thickness comprised between 1 and 5 µm.

5. Implant according to any one of Claims 1 to 4, the density of peaks parameter being less than or equal to 0.5 µm⁻² .

6. Method for manufacturing an implant according to any one of Claims 1 to 5, comprising the following successive steps:
a) supplying an implant body comprising an endosseous surface intended to be implanted into a bone,
b) supplying a solution of an organic material in a solvent, said solution containing a particulate material in suspension,
c) applying the solution to a zone of the endosseous surface of the implant body,
d) evaporating the solvent,
e) heating the implant body to a treatment temperature, the treatment temperature and duration being chosen to be high enough to eliminate the organic material and not high enough to cause the particulate material and the material constituting the implant body to melt.

7. Method according to Claim 6, wherein, before step e), steps c) and d) are repeated several times, preferably at least 50 times.

8. Method according to one of Claims 6 and 7, wherein the organic material is a polymer, preferably a polyethylene glycol, more preferably still, a polyethylene glycol with a molecular weight of around 4000 g/mol.

9. Method according to any one of Claims 6 to 8, wherein, in order to prepare the solution of step b), use is made of a volume of organic material that is 1 to 20 times, preferably 1 to 10 times, more preferably still, 1 to 6 times, higher than the volume of particulate material.

10. Method according to any one of Claims 6 to 9, wherein the particulate material is a powdered ceramic, preferably zirconia, or powdered metal, preferably titanium or a titanium alloy.

11. Method according to any one of Claims 6 to 10, wherein, during step c), the solution is applied to the implant body by spraying.

12. Method according to any one of Claims 6 to 11, wherein, in the solution, the particulate material represents 0.1% to 10% of the volume of the solution, preferably 0.5% to 1%.

13. Method according to any one of Claims 6 to 12, wherein the particulate material has a median particle size less than or equal to 1 µm.

14. Method according to one of Claims 6 to 13, wherein:
- the implant body is made of ceramic, preferably of zirconia, more preferably still, of yttrium-doped or cerium-doped zirconia,
- the particulate material is a powdered ceramic, preferably a powdered zirconia, more preferably still, powdered yttrium-doped or cerium-doped zirconia,
- the organic material is polyethylene glycol.

15. Method according to one of Claims 6 to 14, wherein, during step e), the implant body is heated to a treatment temperature comprised between 600°C and 1600°C, preferably between 1100°C and 1600°C, and more preferably still, between 1300°C and 1600°C.

16. Method according to any one of Claims 6 to 15, wherein the implant body is made of a ceramic, of a metal, or of an alloy of metals, of which the dimensions, during step e), do not vary by more than 3 to 5%.

17. Method according to any one of Claims 6 to 16, wherein the implant body is made of a ceramic, of a metal, or of an alloy of metals, of which the density is at least 95% of its theoretical density.
